(19) **Europäisches Patentamt / European Patent Office / Office européen des brevets**

(11) **EP 3 028 035 B1**

(12) **FASCICULE DE BREVET EUROPEEN**

(45) Date de publication et mention de la délivrance du brevet:
**14.02.2018 Bulletin 2018/07**

(21) Numéro de dépôt: **14762048.8**

(22) Date de dépôt: **01.08.2014**

(51) Int Cl.:
*G01N 21/95* (2006.01)   *H01L 21/66* (2006.01)
*G01N 33/00* (2006.01)

(86) Numéro de dépôt international:
**PCT/FR2014/000179**

(87) Numéro de publication internationale:
**WO 2015/015065 (05.02.2015 Gazette 2015/05)**

(54) **PROCEDE DE LOCALISATION D'UNE PLAQUETTE DANS SON LINGOT**

VERFAHREN ZUR LOKALISIERUNG EINES WAFERS IM INGOT DAVON

METHOD FOR LOCATING A WAFER IN THE INGOT OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.08.2013 FR 1301875**

(43) Date de publication de la demande:
**08.06.2016 Bulletin 2016/23**

(73) Titulaire: **Commissariat à l'Énergie Atomique et aux Énergies Alternatives 75015 Paris (FR)**

(72) Inventeurs:
• **VEIRMAN Jordi**
  **FR-74330 Poisy (FR)**
• **DUBOIS Sébastien**
  **FR-74950 Scionzier (FR)**

(74) Mandataire: **Talbot, Alexandre**
  **Cabinet Hecké**
  **28 Cours Jean Jaures**
  **38000 Grenoble (FR)**

(56) Documents cités:
**JP-A- 2001 076 981    US-B1- 6 482 661**

• J W CORBETT ET AL: "New Oxygen Infrared Bands in Annealed Irradiated Silicon", PHYSICAL REVIEW, vol. 135, no. 5A, 31 août 1964 (1964-08-31), pages A1381-A1385, XP055114040,
• L I MURIN ET AL: "Experimental Evidence of the Oxygen Dimer in Silicon", PHYSICAL REVIEW LETTERS, vol. 80, no. 1, 5 janvier 1998 (1998-01-05), pages 93-96, XP055114044,
• W. WIJARANAKULA: "Formation kinetics of oxygen thermal donors in silicon", APPLIED PHYSICS LETTERS, vol. 59, no. 13, 1 janvier 1991 (1991-01-01), page 1608, XP055114038, ISSN: 0003-6951, DOI: 10.1063/1.106245 cité dans la demande
• KAZUMI WADA: "unified model for formation kinetics of oxygen thermal donors in silicon", PHYSICAL REVIEW, B. CONDENSED MATTER, AMERICAN INSTITUTE OF PHYSICS. NEW YORK, US, vol. 30, no. 10, 15 novembre 1984 (1984-11-15), pages 5884-5895, XP002696153, ISSN: 0163-1829

**Description**

**Domaine technique de l'invention**

L'invention est relative aux techniques de caractérisation des plaquettes semi-conductrices, et plus particulièrement à un procédé pour déterminer l'origine des plaquettes dans le lingot dont elles sont issues.

**État de la technique**

Après qu'un lingot semi-conducteur ait été cristallisé, il est découpé en une pluralité de plaquettes ou substrats. Ces plaquettes sont ensuite vendues par lot, pour servir à la fabrication de circuits intégrés ou de cellules photovoltaïques.
Les fabricants de tels composants mettent en place des solutions d'identification pour le suivi des plaquettes tout au long des processus de fabrication. Ces solutions leur permettent, avec un outil informatique de gestion approprié, d'enregistrer des informations concernant la plaquette ou le lot correspondant, par exemple l'historique des étapes de fabrication.
En général, les fabricants de circuits intégrés ou de cellules photovoltaïques n'ont pas connaissance de la position originelle des plaquettes dans leur lingot. Cette information n'est pas communiquée par le fabriquant du lingot, essentiellement pour des raisons économiques et de traçabilité. Pourtant, cette information est cruciale pour l'utilisateur des plaquettes, car les défauts et les impuretés ne sont pas répartis uniformément dans un lingot semi-conducteur. Par exemple, la partie haute du lingot, solidifiée en premier lors du tirage du lingot, contient une grande quantité d'oxygène. La partie basse du lingot, solidifiée en dernier, est riche en impuretés métalliques. Il en résulte que les propriétés électriques et mécaniques des plaquettes varient considérablement d'une portion à l'autre du lingot.
A l'heure actuelle, la position initiale des plaquettes dans le lingot ne peut pas être déterminée sans indications de la part du fabricant du lingot. Même lorsqu'on dispose d'informations, celles-ci peuvent être partielles, voire erronées. Le document JP2001076981 divulgue un procédé de détermination de la position originelle d'une plaquette dans un lingot en matériau semi-conducteur. Une marque en face avant et en face arrière permet d'identifier la position du lingot lors de la découpe du lingot.

**Résumé de l'invention**

Il existe donc un besoin de prévoir un procédé pour déterminer, a posteriori, la position originelle d'une plaquette dans un lingot en matériau semi-conducteur.
Selon l'invention, on tend à satisfaire ce besoin en prévoyant les étapes suivantes :

- mesurer la concentration en oxygène interstitiel en une zone de la plaquette ;

- mesurer la concentration en donneurs thermiques formés dans ladite zone de la plaquette pendant une précédente solidification du lingot ;

- déterminer la durée effective d'un recuit de formation des donneurs thermiques, subi par la plaquette lors de la solidification du lingot, à partir de la concentration en donneurs thermiques et de la concentration en oxygène interstitiel ; et

- déterminer la position originelle de la plaquette dans le lingot à partir de la durée effective.

Selon un développement de l'invention, la concentration en donneurs thermiques est déterminée à partir de deux valeurs de résistivité, mesurées dans ladite zone de la plaquette avant et après un traitement thermique visant à détruire les donneurs thermiques.
Selon un autre développement, compatible avec le précédent, la concentration en oxygène interstitiel est déterminée à partir de deux valeurs de résistivité, mesurées dans ladite zone de la plaquette avant et après un recuit additionnel de formation des donneurs thermiques.
Dans un mode de réalisation préférentiel de l'invention, le traitement thermique de destruction des donneurs thermiques est réalisé après le recuit additionnel de formation des donneurs thermiques.
La position originelle de la plaquette peut être déterminée à l'aide d'un abaque, établi d'après les étapes suivantes :

- sélectionner une pluralité de plaquettes provenant d'un même lingot ;

- déterminer, pour chaque plaquette, une valeur de la durée effective ;

- placer sur l'abaque deux points correspondant aux valeurs minimale et maximale de durée effective parmi l'ensemble des valeurs de durée effective préalablement déterminées ; et

- tracer une droite entre les deux points.

Les valeurs minimale et maximale de durée effective sont, de préférence, associées à des positions originelles valant 5 % et 85 % de la hauteur totale du lingot.

## Description sommaire des dessins

D'autres avantages et caractéristiques ressortiront plus clairement de la description qui va suivre de modes particuliers de réalisation donnés à titre d'exemples non limitatifs et illustrés à l'aide des dessins annexés, dans lesquels :

- la figure 1 représente l'évolution de la température d'une portion en tête de lingot et d'une portion en pied de lingot, lors de sa cristallisation ;

- la figure 2 représente des étapes d'un procédé visant à déterminer la position originelle d'une plaquette semi-conductrice dans son lingot parent ;

- la figure 3 est un abaque donnant la position relative d'une plaquette dans son lingot, en fonction de la durée équivalente de recuit calculée à l'étape F3 de la figure 2 ;

- la figure 4 représente un mode de mise en oeuvre préférentiel de l'étape F1 de la figure 2 ; et

- la figure 5 représente un mode de mise en oeuvre préférentiel de l'étape F2 de la figure 2.

## Description d'un mode de réalisation préféré de l'invention

Les lingots en silicium obtenus par les techniques classiques de cristallisation, par exemple le procédé Czochralski pour le silicium monocristallin et le procédé Brigman pour le silicium multicristallin, contiennent de l'oxygène. Les atomes d'oxygène occupent notamment des positions interstitielles dans le(s) réseau(x) cristallin(s).

Au cours de la cristallisation du silicium, la température du lingot diminue lentement, passant de 1414 °C (température de fusion du silicium) à la température ambiante (environ 25 °C). Or, entre 350 °C et 500 °C, l'oxygène en position interstitielle forme des agglomérats appelés donneurs thermiques (DT). Ces donneurs thermiques ont la particularité de générer des électrons libres. Ils ont donc un caractère dopant et influent sur les propriétés électriques du silicium.

Il existe donc des donneurs thermiques dans un lingot à l'issu de sa solidification, et par conséquent, dans les plaquettes qui sont issues de ce lingot. La concentration en donneurs thermiques n'est cependant pas uniforme sur la hauteur du lingot. Cela est dû au fait que les différentes portions du lingot ne sont pas refroidies à la même vitesse et qu'elles ne contiennent pas la même teneur en oxygène interstitiel.

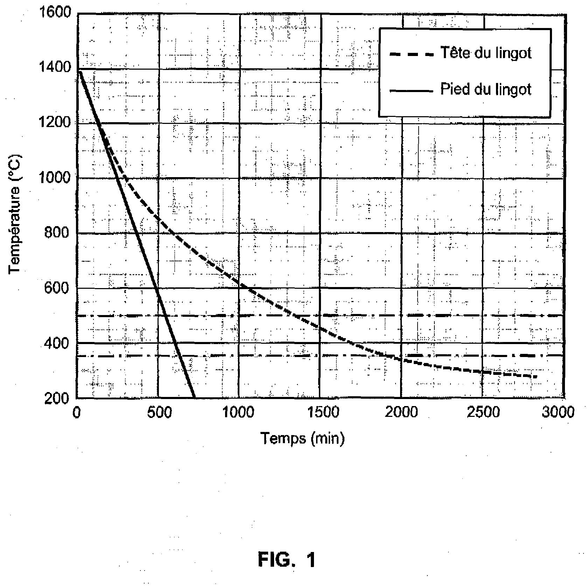

La figure 1 représente les profils de température des extrémités haute et basse d'un lingot en silicium, lors du tirage selon la méthode Czochralski. La courbe de température de l'extrémité haute, appelée tête du lingot, est représentée en traits pointillés, tandis que la courbe de température de l'extrémité basse, appelée pied du lingot, est représentée en trait plein.

Comme cela illustré par la figure 1, la température en tête de lingot diminue plus lentement que la température en pied de lingot au cours du temps. En effet, après la solidification de la partie haute du lingot, celle-ci reste en échange thermique avec le bain de silicium en fusion pendant toute la durée du tirage, par l'intermédiaire du corps de lingot en formation. Par contre, à la fin du tirage, la portion basse se solidifie puis le lingot est retiré du bain, ce qui explique pourquoi la température du pied du lingot diminue plus rapidement.

A cause de ces différentes cinétiques de refroidissement, la tête du lingot passe davantage de temps que le pied du lingot dans la plage de températures correspondant à la formation des donneurs thermiques. Dans l'exemple de lingot de la figure 1, la tête du lingot passe plus de 600 minutes dans la plage 350 °C-500 °C (représentée en trait mixte), alors que le pied du lingot y reste seulement 80 minutes.

Les portions intermédiaires du lingot en silicium, c'est-à-dire celles situées entre la tête et le pied, voient leur température baisser à un rythme compris entre les deux courbes. Par conséquent, la durée pendant laquelle ces portions ont une température comprise dans la plage 350 °C-500 °C se situe entre 80 min et 600 min.

Ainsi, la durée effective $t_{eff}$ de formation des donneurs thermiques pendant la solidification d'une portion du lingot dépend de la position h de cette portion dans le lingot. On constate en outre que la fonction $t_{eff}$(h) est strictement croissante ou décroissante, selon qu'on considère le pied ou la tête comme l'origine des positions dans le lingot. Autrement dit, il existe une relation de bijection entre la durée $t_{eff}$ et la position h dans le lingot : à chaque portion du lingot correspond une durée $t_{eff}$ unique.

Ce constat est, ci-après, mis en application pour déterminer l'origine dans le lingot d'une plaquette en silicium. La durée effective $t_{eff}$ de formation des donneurs thermiques (entre 350 °C et 500 °C) est déterminée à partir de relevés de la concentration en oxygène interstitiel et de la concentration en donneurs thermiques présents initialement dans la plaquette.

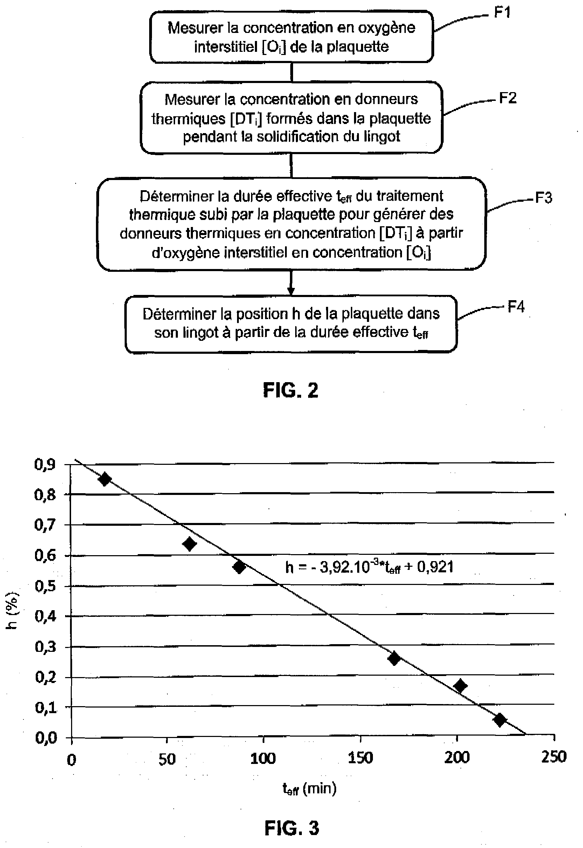

La figure 2 représente des étapes F1 à F4 de ce procédé de localisation des plaquettes en silicium.

En F1, la concentration en oxygène interstitiel, notée $[O_i]$, est déterminée en au moins une zone de la plaquette. La concentration $[O_i]$ peut notamment être mesurée par spectrométrie de masse à ionisation secondaire (SIMS) ou par spectroscopie infrarouge à transformée de Fourrier (FTIR). Cette dernière technique permet de mesurer l'absorption d'un rayonnement infrarouge dans le matériau semi-conducteur, en fonction de la longueur d'onde de ce rayonnement. Comme l'oxygène interstitiel contribue à cette absorption, il est possible de déduire la concentration $[O_i]$ de la mesure d'absorption.

L'étape F2 consiste à déterminer la concentration initiale en donneurs thermiques $[DT]_i$ dans la ou les mêmes zones de la plaquette. Comme indiqué précédemment, ces donneurs thermiques sont apparus lors du refroidissement du lingot, entre 350 °C et 500 °C.

A nouveau, la technique FTIR peut être utilisée pour mesurer la concentration $[DT]_i$, car les donneurs thermiques ont également une influence sur l'absorption du rayonnement infrarouge. Comme l'oxygène, les donneurs thermiques font apparaître des pics caractéristiques dans le spectre d'absorption. A chaque pic correspond une famille de donneurs thermiques (16 familles répertoriées au total, toutes ayant le même comportement donneur). La teneur en donneurs thermiques $[DT]_i$ est calculée en convertissant l'amplitude des pics d'absorption et en additionnant les valeurs obtenues.

Ainsi, à l'issue de l'étape F2, on dispose d'un ou plusieurs couples de valeurs $[O_i]$ et $[DT]_i$.

A l'étape F3, on détermine la durée effective $t_{eff}$ du traitement thermique entre 350 °C et 500 °C ayant permis la formation des donneurs thermiques en concentration $[DT]_i$, à partir de la teneur en oxygène $[O_i]$.

La durée effective $t_{eff}$ est, de préférence, calculée à partir des valeurs de $[O_i]$ et $[DT]_i$ mesurées aux étapes F1 et F2, à l'aide d'une relation tirée de l'article [« Formation kinetics of oxygen thermal donors in silicon », Wijaranakula C.A. et al., Appl. Phys. Lett. 59 (13), pp. 1608, 1991]. Cet article décrit la cinétique de formation des donneurs thermiques dans le silicium par un recuit à 450 °C.

On considère alors que la durée $t_{eff}$ est équivalente à la durée d'un recuit à 450 °C qu'il aurait fallu employer pour obtenir une concentration en donneurs thermiques égale à $[DT]_i$, à partir d'une concentration en oxygène égale à $[O_i]$.

D'après l'article susmentionné, la concentration $[DT]_i$, la concentration $[O_i]$ et la durée t du recuit à 450 °C sont liées par la relation suivante :

$$[DT]_i = 4,51.10^{-52} \times \left( [O_i] \left( 1 + \frac{2}{3} D_o \times t \times [O_i]^{2/3} \right)^{-3/2} \right)^{3,45} \times t^{1,02} \qquad (1)$$

avec $D_o$ le coefficient de diffusion de l'oxygène interstitiel à 450 °C ($D_o$ = 3,5.10$^{-19}$ cm$^2$/s).

La durée équivalente t ainsi calculée constitue une bonne approximation de la durée effective $t_{eff}$, soit le temps qu'a passé la plaquette dans la plage 350 °C-500 °C lors de la solidification du lingot.

Pour le calcul de la durée $t_{eff}$, la relation (1) ci-dessus est privilégiée car la température de 450 °C est celle à laquelle la cinétique de formation des donneurs thermique est la mieux documentée. Le recuit à 450 °C a fait l'objet de nombreuses études, car il constitue un bon compromis entre la vitesse de formation des donneurs thermiques et la concentration maximale obtenue.

Alternativement, la durée effective $t_{eff}$ peut être déterminée à l'aide d'abaques donnant la concentration en donneurs thermiques $[DT]_i$ en fonction de la durée t du recuit à 450 °C, pour différentes valeurs de la concentration en oxygène $[O_i]$.

On peut également calculer la durée effective $t_{eff}$ en considérant une température de référence différente de 450 °C. La relation (1) et les abaques seront alors adaptés notamment grâce aux enseignements de l'article [« Effect of oxygen concentration on the kinetics of thermal donor formation in silicon at temperatures between 350 and 500 °C », Londos C.A. et al., Appl. Phys. Lett. 62 (13), pp. 1525, 1993]. Cet article décrit également la cinétique de formation des donneurs thermiques dans le silicium, mais pour des températures de recuit comprises entre 350 °C et 500 °C.

Enfin, lorsqu'on dispose de plusieurs couples de valeurs et $[DT]_i$ pour une même plaquette de silicium, on peut calculer une durée moyenne $t_{moy}$ de recuit à 450 °C. Cette moyenne constituera alors un meilleur indicateur de la durée effective $t_{eff}$.

L'étape F4 de la figure 2 consiste à déterminer la position originelle h de la plaquette dans son lingot, connaissant la durée effective $t_{eff}$. Pour cela, on met en application la relation de bijection entre les grandeurs $t_{eff}$ et h.

Avantageusement, la position h est déterminée à l'aide d'un abaque représentatif de la fonction $h(t_{eff})$. Un tel abaque permet de lire facilement et rapidement la position h du lingot correspondant à une valeur de durée effective $t_{eff}$ calculée à l'étape F3. De préférence, cet abaque est construit en faisant l'hypothèse que la fonction $h(t_{eff})$ est une fonction affine. La courbe de l'abaque est donc une droite.

Pour établir l'abaque, on dispose d'une pluralité de plaquettes provenant d'un même lingot. Les plaquettes sont, de préférence, prélevées aléatoirement dans les lots correspondant au lingot. En outre, comme c'est souvent le cas des substrats à destination de l'industrie photovoltaïque, les plaquettes du lingot n'ont pas subi de traitement thermique visant à détruire les donneurs thermiques.

Les concentrations $[O_i]$ et $[DT]_i$ sont d'abord mesurées pour chaque plaquette, puis les différentes valeurs de durée effective $t_{eff}$ sont calculées. Ces étapes peuvent être réalisées comme décrit ci-dessus, en relation avec la figure 1 (étapes F1-F3).

Parmi l'ensemble des valeurs de durée effective $t_{eff}$, on suppose que la plus élevée, notée $t_{eff(max)}$, correspond à la partie cristallisée en premier, soit le haut du lingot (tête), et que la plus faible, $t_{eff(min)}$, correspond à la partie basse (pied), cristallisée en dernier.

A l'issue du tirage, les fabricants de lingots ont pour habitude d'écarter les portions inférieure et supérieure situées aux extrémités du lingot, car elles contiennent d'importantes quantités d'impuretés. Elles sont inutilisables et par conséquent mises au rebut. En général, ces portions inférieure et supérieure représentent respectivement 15 % et 5 % de la hauteur totale du lingot.

Ainsi, la valeur $t_{eff(max)}$ de durée effective correspond à une position dans le lingot d'environ 5 % de la hauteur totale, et la plus faible valeur de durée effective $t_{eff}$ correspond à une position dans le lingot d'environ 85 % (l'origine de cette hauteur relative correspondant à la tête du lingot).

Partant de cette hypothèse, on peut tracer la droite $h(t_{eff})$ sur l'abaque en plaçant deux points, l'un à 5 %, l'autre à 85 % de la hauteur totale du lingot. L'abaque ainsi obtenu peut être utilisé pour tous les lingots qui ont été cristallisés dans des conditions similaires. Un abaque est avantageusement construit pour chaque fournisseur de lingots et pour chaque procédé de tirage (Czochralski, Brigman...). Pour une même technique de tirage et un même fournisseur, plusieurs abaques peuvent être nécessaires, si les conditions opératoires ont variées suffisamment pour modifier les profils de refroidissement des lingots.

A titre d'exemple, la durée effective $t_{eff}$ a été mesurée pour 6 plaques de silicium monocristallin Czochralski provenant d'un même lingot. Les résultats sont donnés dans le tableau 1 ci-dessous :

**Tableau 1**

| Plaquette | $t_{eff}$ (min) |
|-----------|-----------------|
| 1 | 168 |
| 2 | 202 |
| 3 | 222 |
| 4 | 62 |
| 5 | 88 |
| 6 | 18 |

Les valeurs maximale et minimale de la durée effective $t_{eff}$ sont obtenues respectivement pour la plaquette n° 3 et la plaquette n° 6. Les plaquettes 3 et 6 correspondent donc aux fractions situées respectivement à 5 % et 85 % de la hauteur du lingot.

La figure 3 est un graphique de la position relative h dans le lingot en fonction de la durée effective $t_{eff}$. Les deux valeurs de $t_{eff}$ obtenues précédemment permettent de placer deux points (aux coordonnées {$t_{eff}$ = 18 ; h = 0,85} et {$t_{eff}$ = 222 ; h = 0,05}. Ces deux points extrêmes permettent de tracer une droite d'équation $h = -3{,}92 \cdot 10^{-3} \cdot t_{eff} + 0{,}921$.

Cette droite ou son équation permettra de calculer la position originelle h de toute plaquette ultérieure, issue du même lingot ou d'un lingot équivalent, à partir de la valeur de durée effective calculée pour cette plaquette.

Avec les autres valeurs de $t_{eff}$ du tableau 1, il est possible de vérifier que l'hypothèse d'une fonction affine est justifiée. Pour cela, il faut néanmoins disposer d'informations provenant du fabricant du lingot, qui permettent de déterminer précisément les hauteurs des plaquettes 1, 2, 4 et 5.

D'après les données du fournisseur, la position h des plaquettes 1, 2, 4 et 5 vaut respectivement 0,253, 0,162, 0,636 et 0,560. En reportant ces valeurs sur l'abaque de la figure 3, on constate que les points coïncident avec la droite d'équation h = - 3,92.10-3*$t_{eff}$ + 0,921. Par conséquent, l'hypothèse selon laquelle h($t_{eff}$) est une fonction affine est vérifiée.

Cette méthode de construction d'un abaque est rapide et facile à mettre en oeuvre. Pour davantage de précision, il est préférable de sélectionner un grand nombre de plaquettes afin de s'approcher au plus près des valeurs maximale et minimale correspondant effectivement aux extrémités du lingot. Sur la figure 1, il existe un facteur d'environ 10 entre la durée effective du pied du lingot et celle de la tête du lingot. En pratique, le nombre de plaquettes mesurées sera considéré comme suffisant dès lors qu'un rapport $t_{eff(max)}/t_{eff(min)}$ d'environ 10 est atteint.

La technique FTIR, utilisée aux étapes F1 et F2 pour la mesure des concentrations [$O_i$] et [DT]$_i$, requiert une plaquette avec une épaisseur supérieure à 300 $\mu$m, un état de surface poli optique (finition « miroir ») et des surfaces parfaitement parallèles. Par conséquent, cette technique est inadaptée à l'industrie photovoltaïque, qui utilise des substrats bruts de sciage et d'une épaisseur inférieure à 200 $\mu$m. En outre, une mesure FTIR pour déterminer [DT]$_i$ est généralement réalisée à très basse température (4 K), afin de réduire le bruit de fond causé par l'absorption des phonons. Elle est donc difficilement applicable à grande échelle.

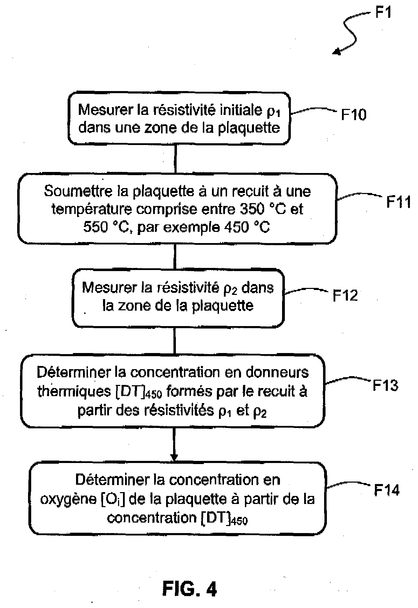

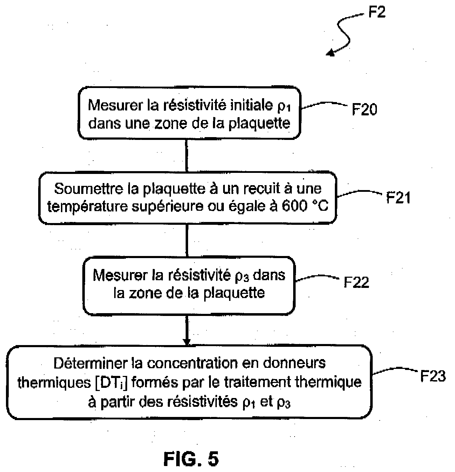

Ainsi, pour remédier aux inconvénients de la technique FTIR, des techniques alternatives ont été mises en oeuvre pour déterminer les concentrations [$O_i$] et [DT]$_i$. Ces variantes des étapes F1 et F2 sont décrites ci-après, en relation avec les figures 4 et 5.

La figure 4 représente donc une autre technique de détermination de la concentration en oxygène interstitiel [$O_i$]. Cette technique, basée également sur la formation de donneurs thermiques, a été décrite en détail dans le brevet français FR2964459, pour la cartographie en oxygène d'une plaque de silicium.

Sur la figure 4, l'étape F1 de détermination de la concentration [$O_i$] est décomposée en plusieurs sous-étapes F10 à F14. En F10, on mesure la résistivité électrique initiale $\rho_1$ dans une zone de la plaquette en silicium. La résistivité $\rho_1$ tient compte des donneurs thermiques et des autres dopants (par exemple des atomes de bore dans le cas d'une plaquette dopée de type p), tous présents à l'origine dans la plaquette (en concentration [DT]$_i$ pour les donneurs thermiques) :

$$\rho_1 = \frac{1}{q \times \mu([B],[DT]_i) \times ([B] - 2[DT]_i)}$$

Dans l'expression ci-dessus, q est la charge élémentaire (q = 1,6.10-19 C) et [B] est la concentration en dopants (ex. bore). $\mu$ désigne la mobilité des porteurs de charge qui, d'après l'enseignement du brevet FR2964459, dépend de la concentration [DT]$_i$, en plus de la concentration en dopants [B].

La plaquette est ensuite soumise à un recuit, de manière à former des donneurs thermiques supplémentaires (sous-étape F11). La température de ce recuit est, de préférence, comprise entre 350 °C et 500 °C, par exemple 450 °C. Sa durée peut varier d'une dizaine de minutes à quelques heures. Ce recuit vise à former de nouveaux donneurs thermiques, en plus de ceux formés pendant la solidification du lingot.

Après le recuit F11, une nouvelle valeur $\rho_2$ de la résistivité électrique est mesurée, de préférence dans la même zone de la plaquette (sous-étape F12). Les résistivités $\rho_1$ et $\rho_2$ sont, par exemple, mesurées par la méthode des quatre pointes, la méthode de Van der Pauw ou encore par courant de Foucault.

La variation de la résistivité $\rho_2$-$\rho_1$ est attribuable à la formation des nouveaux donneurs thermiques, lors du recuit F11. Les valeurs de résistivité $\rho_1$ et $\rho_2$ permettent de calculer, lors d'une sous-étape F13, la concentration en donneurs thermiques [DT]$_{450}$.

Plus particulièrement, la résistivité $\rho_2$ s'écrit :

$$\rho_2 = \frac{1}{q \times \mu([B],[DT]_i + [DT]_{450}) \times ([B] - 2[DT]_i - 2[DT]_{450})}$$

Dans cette expression, par rapport à celle de la résistivité $\rho_1$, la concentration [DT]$_{450}$ en donneurs thermiques formés pendant le recuit F11 s'ajoute à la concentration initiale [DT]$_i$ en donneurs thermiques.

Comme dans le brevet FR2964459, on peut supposer que la concentration initiale [DT]$_i$ est largement inférieure à la concentration en donneurs thermiques [DT]$_{450}$ formés pendant le recuit F11 et aux teneurs en dopants ([DT]$_i$ << [DT]$_{450}$ et, dans l'exemple du bore comme dopant, [DT]$_i$ << [B]). La concentration en dopants [B] est alors tirée de la valeur de résistivité $\rho_1$ mesurée avant le recuit F11, et la concentration [DT]$_{450}$ est calculée à partir de la valeur de résistivité $\rho_2$, connaissant la concentration en dopants.

On peut aussi prendre en compte dans le calcul de [DT]$_{450}$ la quantité initiale [DT]$_i$ de donneurs thermiques qu'on aura mesurée à l'étape F2. Ce mode de calcul est le plus précis et sera donc privilégié.

Enfin, en F14, la concentration en oxygène $[O_i]$ dans la plaquette de silicium est déterminée à partir de la concentration $[DT]_{450}$ et de la durée du recuit F11. Une équation mathématique, par exemple la relation (1) ci-dessus, ou des abaques donnant la concentration en oxygène pour différentes durées et températures de recuit, peuvent être utilisés à cet effet.

Pendant la solidification du lingot, entre 500 °C et 350 °C, et pendant le recuit F11, la concentration en oxygène interstitiel ne varie pratiquement pas car la durée effective $t_{eff}$ et la durée du recuit sont faibles. La valeur mesurée en F14 représente donc bien la quantité d'oxygène présent initialement dans la portion du lingot (i.e. avant son refroidissement).

La figure 5 représente une variante de mise en oeuvre de l'étape F2 visant à déterminer la concentration en donneurs thermiques $[DT]_i$. Cette variante reprend le principe d'une variation de résistivité causée par les donneurs thermiques, comme cela a été décrit en relation avec la figure 4. Elle comprend donc des étapes F20 et F22 de mesure de résistivité, respectivement avant (résistivité $\rho_1$) et après (résistivité $\rho_3$) une étape de recuit F21. Toutefois, à la différence du recuit F11 de la figure 4, le recuit F21 ne vise pas à former des donneurs thermiques supplémentaires, mais au contraire à détruire ceux présents initialement dans la plaquette. Le recuit F21 est réalisé à une température supérieure ou égale à 600 °C, par exemple pendant 30 minutes.

La valeur $\rho_3$ mesurée après le recuit F21 est la résistivité de la plaquette dépourvue de donneurs thermiques. Par conséquent, elle ne dépend que des concentrations en dopants. Dans l'exemple du bore comme seul dopant, son expression est la suivante :

$$\rho_3 = \frac{1}{q \times \mu([B]) \times [B]}$$

La valeur $\rho_1$ mesurée avant le recuit F21 représente l'état de la plaquette telle qu'elle est livrée par le fabricant de lingots. Comme indiqué précédemment, elle tient compte des dopants et des donneurs thermiques formés pendant la solidification du lingot, en concentration $[DT]_i$.

Ainsi, lors d'une étape F23, les teneurs en dopants sont calculées à partir de la résistivité $\rho_3$, puis la concentration $[DT]_i$ est calculée à partir de la résistivité $\rho_1$ et des concentrations en dopants.

Les techniques de mesure des figures 4 et 5 sont applicables quels que soient l'épaisseur des plaquettes et leur état de surface. Elles nécessitent seulement des mesures de résistivité et des traitements thermiques, qui sont des étapes faciles à réaliser et non-destructives. Ces deux techniques sont donc adaptées à l'industrie du semi-conducteur, et conviennent plus particulièrement aux substrats en silicium de qualité photovoltaïque.

Les étapes F1 et F2 des figures 4 et 5 peuvent être réalisées indépendamment l'une de l'autre. Elles peuvent aussi être utilisées conjointement.

Lorsque les étapes F1 et F2 des figures 4 et 5 s'enchaînent dans cet ordre (comme suggéré par la figure 2), l'étape F20 de la figure 5 peut être supprimée, car on dispose déjà d'une valeur de référence : la valeur $\rho_1$ mesurée au départ, en F10. Après avoir déterminé la concentration $[O_i]$ en F14, on procédera alors directement au recuit F21 puis à la mesure d'une troisième valeur de résistivité $\rho_3$ (en F22). Enfin, la concentration en donneurs thermiques initiale $[DT]_i$ sera calculée à partir des résistivités $\rho_3$ ($[DT]=0$) et $\rho_1$ ($[DT]=[DT]_i$).

En outre, lorsque l'étape F2 succède à l'étape F1, le recuit F21 de destruction des donneurs thermiques est réalisé après le recuit F11 de formation des donneurs thermiques. Il n'y aura donc plus de donneurs thermiques dans la plaquette à l'issue du procédé. Cet enchainement d'étapes est donc avantageux si l'on veut s'affranchir des donneurs thermiques, pour les étapes ultérieures de fabrication des composants électroniques ou photovoltaïques.

Si l'on souhaite au contraire profiter des propriétés électriques conférées par les donneurs thermiques, on pourra mesurer la concentration initiale $[DT]_i$ en donneurs thermiques de la plaquette (étape F2-Fig.5) avant de mesurer la concentration en oxygène interstitiel $[O_i]$ (étape F1-Fig.4). Alors, on commencera pas détruire les donneurs thermiques initiaux, avant d'en créer de nouveaux. On pourra, comme précédemment, ne réaliser que trois mesures de résistivité : résistivité initiale $\rho_1$ avant le recuit F20 ($[DT] = [DT]_i$), résistivité $\rho_3$ après le recuit F20 ($[DT] = 0$), et résistivité $\rho_2$ après le recuit F10 ($[DT] = [DT]_{450}$).

Autrement dit, le procédé de localisation des plaquettes n'est limité à un aucun ordre des étapes F1 et F2.

Comme indiqué plus haut, il est préférable de connaître la concentration initiale $[DT]_i$ en donneurs thermiques avant de mesurer la concentration en oxygène interstitiel $[O_i]$. La mesure de $[O_i]$ est en effet plus précise, en prenant en compte les donneurs thermiques initiaux dans le calcul de la concentration $[DT]_{450}$ (étape F13 - à partir de l'expression de $\rho_2$). Réaliser le recuit à haute température F21 après le recuit F11 à 450 °C permet aussi d'obtenir une meilleure précision sur la mesure de $[O_i]$. Dans l'ordre inverse, le recuit F21 peut influencer la cinétique de formation des donneurs thermiques lors du recuit suivant F11 et donc fausser la mesure la concentration en oxygène interstitiel $[O_i]$.

Ainsi, dans un mode de réalisation préférentiel, les étapes des figures 4 et 5 sont enchevêtrées de sorte que le recuit F21 succède au recuit F11 et que la concentration $[DT]_i$ est déterminée avant la concentration $[O_I]$. L'ordre des étapes est avantageusement le suivant :

- F10 (=F20) : mesure de la résistivité $\rho_1$ ($[DT] = [DT]_i$) ;

- F11 : recuit à 350 °C-500 °C ;

- F12 : mesure de la résistivité $\rho_2$ ($[DT] = [DT]_i + [DT]_{450}$) ;

- F21 : recuit à 600 °C ou plus ;

- F22 : mesure de la résistivité $\rho_3$ ($[DT] = 0$) ;

- F23 : calcul de $[DT]_i$ à partir de $\rho_1$ et $\rho_3$ ;

- F13 : calcul de $[DT]_{450}$ à partir de $\rho_1$, $\rho_2$ et $[DT]_i$ ; et

- F14 : détermination de $[O_i]$ à partir de $[DT]_{450}$.

Bien que ce procédé ait été décrit en relation avec une plaquette en silicium monocristallin Cz, il pourrait être appliqué à d'autres formes de silicium (structure quasi-monocristalline ou multicristalline) et d'autres matériaux semi-conducteurs. Le germanium est, par exemple, un candidat potentiel, car des donneurs thermiques à base d'oxygène sont également formés lors de sa cristallisation. Enfin, le lingot dont sont issues les plaquettes peut être cristallisé selon des techniques de tirage autres que les procédés Czochralski ou Brigman, pourvu que les différentes portions du lingot subissent des cinétiques de refroidissements distinctes.

## Revendications

1.  Procédé pour déterminer la position originelle d'une plaquette dans un lingot en matériau semi-conducteur, comprenant les étapes suivantes :

    - mesurer (F1) la concentration en oxygène interstitiel ($[O_i]$) en une zone de la plaquette ;
    - mesurer (F2) la concentration en donneurs thermiques ($[DT_i]$) formés dans ladite zone de la plaquette pendant une précédente solidification du lingot ;
    - déterminer (F3) la durée effective ($t_{eff}$) d'un recuit de formation des donneurs thermiques, subi par la plaquette lors de la solidification du lingot, à partir de la concentration en donneurs thermiques ($[DT_i]$) et de la concentration en oxygène interstitiel ($[O_i]$) ; et
    - déterminer (F4) la position originelle de la plaquette dans le lingot à partir de la durée effective ($t_{eff}$).

2.  Procédé selon la revendication 1, dans lequel la concentration en donneurs thermiques ($[DT_i]$) est déterminée à partir de deux valeurs ($\rho_1$, $\rho_3$) de résistivité, mesurées dans ladite zone de la plaquette avant (F20) et après (F22) un traitement thermique (F21) visant à détruire les donneurs thermiques.

3.  Procédé selon la revendication 2, dans lequel la concentration en oxygène interstitiel ($[O_i]$) est déterminée à partir de deux valeurs ($\rho_1$, $\rho_2$) de résistivité, mesurées dans ladite zone de la plaquette avant (F10) et après (F12) un recuit additionnel (F11) de formation des donneurs thermiques.

4.  Procédé selon la revendication 3, dans lequel le traitement thermique (F21) de destruction des donneurs thermiques est réalisé après le recuit additionnel (F11) de formation des donneurs thermiques.

5.  Procédé selon l'une quelconque des revendications 1 à 4, dans lequel la position originelle (h) de la plaquette est déterminée à l'aide d'un abaque ($h((t_{eff})$), établi d'après les étapes suivantes :

    - sélectionner une pluralité de plaquettes provenant d'un même lingot ;
    - déterminer (F1, F2, F3), pour chaque plaquette, une valeur de la durée effective ($t_{eff}$) ;
    - placer sur l'abaque deux points correspondant aux valeurs minimale ($t_{eff(min)}$) et maximale ($t_{eff(max)}$) de durée effective parmi l'ensemble des valeurs de durée effective préalablement déterminées ; et

- tracer une droite entre les deux points.

**6.** Procédé selon la revendication 5, dans lequel les valeurs minimale et maximale ($t_{eff(min)}$, $t_{eff(max)}$) de durée effective sont associées à des positions originelles (h) valant 5 % et 85 % de la hauteur totale du lingot.

**Patentansprüche**

**1.** Verfahren zur Bestimmung der ursprünglichen Position eines Wafers in einem Ingot aus Halbleiter-Material, welches die folgenden Verfahrensschritte umfasst:

- Messen (F1) der Konzentration an interstitiellem Sauerstoff ($[O_i]$) innerhalb eines Bereichs des Wafers;
- Messen (F2) der Konzentration an thermischen Donatoren ($[DT_i]$), die sich in dem genannten Bereich des Wafers während eines vorausgegangenen Erstarren des Ingots gebildet haben;
- Bestimmen (F3) der tatsächlichen Dauer ($t_{eff}$) eines Glühens mit Bildung der thermischen Donatoren, dem der Wafer beim Erstarren des Ingots ausgesetzt war, aus der Konzentration an thermischen Donatoren ($[DT_i]$) und der Konzentration an interstitiellem Sauerstoff ($[O_i]$), und
- Bestimmen (F4) der ursprünglichen Position des Wafers in dem Ingot aus der tatsächlichen Dauer ($t_{eff}$).

**2.** Verfahren nach Anspruch 1, wobei die Konzentration an thermischen Donatoren ($[DT_i]$) aus zwei Leitungswiderstandswerten ($p_1$, $p_3$) bestimmt wird, die in dem genannten Bereich des Wafers vor (F20) und nach (F22) einer auf die Zerstörung der thermischen Donatoren gerichteten Wärmebehandlung (F21) gemessen werden.

**3.** Verfahren nach Anspruch 2, wobei die Konzentration an interstitiellem Sauerstoff ($[O_i]$) aus zwei Leitungswiderstandswerten ($p_1$, $p_2$) bestimmt wird, die in dem genannten Bereich des Wafers vor (F10) und nach (F12) einem zusätzlichen Glühen (F11) zur Bildung der thermischen Donatoren gemessen werden.

**4.** Verfahren nach Anspruch 3, wobei die Wärmebehandlung (F21) zur Zerstörung der thermischen Donatoren nach dem zusätzlichen Glühen (F11) zur Bildung der thermischen Donatoren durchgeführt wird.

**5.** Verfahren nach einem der Ansprüche 1 bis 4, wobei die ursprüngliche Position (h) des Wafers mittels eines Diagramms ($h((t_{eff}))$) bestimmt wird, das gemäß den folgenden Verfahrensschritten erstellt wird:

- Auswählen einer Vielzahl von Wafern, die aus einem und demselben Ingot stammen;
- Bestimmen eines Werts der tatsächlichen Dauer ($t_{eff}$) für jeden Wafer;
- Eintragen von zwei Punkten, die dem zuvor bestimmten Minimalwert ($t_{eff(min)}$) und Maximalwert ($t_{eff(max)}$) der tatsächlichen Dauer entsprechen, in das Diagramm; und
- Ziehen einer Geraden zwischen den beiden Punkten.

**6.** Verfahren nach Anspruch 5, wobei der Minimalwert und Maximalwert ($t_{eff(min)}$, $t_{eff(max)}$) der tatsächlichen Dauer ursprünglichen Positionen (h) zugeordnet werden, die mit 5 % bzw. 85 % der Gesamthöhe des Ingots bestimmt sind.

**Claims**

**1.** A method for determining the original position of a wafer in an ingot made from semiconductor material, comprising the following steps:

- measuring (F1) the interstitial oxygen concentration ($[O_i]$) in one area of the wafer;
- measuring (F2) the concentration of thermal donors ($[TD_i]$) formed in said area of the wafer during a previous solidification of the ingot;
- determining (F3) the effective time ($t_{eff}$) of a thermal donor formation anneal undergone by the wafer when solidification of the ingot took place from the thermal donor concentration ($[TD_i]$) and the interstitial oxygen concentration ($[O_i]$); and
- determining (F4) the original position of the wafer in the ingot from the effective time ($t_{eff}$).

**2.** The method according to claim 1, wherein the thermal donor concentration ($[TD_i]$) is determined from two resistivity values ($\rho_1$, $\rho_3$) measured in said area of the wafer before (F20) and after (F22) heat treatment (F21) for the purposes of destroying the thermal donors.

3. The method according to claim 2, wherein the interstitial oxygen concentration ($[O_i]$) is determined from two resistivity values ($\rho_1$, $\rho_2$) measured in said area of the wafer before (F10) and after (F12) an additional thermal donor formation anneal (F11).

4. The method according to claim 3, wherein the heat treatment (F21) for the purpose of destroying the thermal donors is performed after the additional thermal donor formation anneal (F11).

5. The method according to any one of claims 1 to 4, wherein the original position (h) of the wafer is determined by means of an abacus ($h((t_{eff})$)), established according to the following steps:

- selecting a plurality of wafers originating from the same ingot;
- determining (F1, F2, F3) an effective time value ($t_{eff}$) for each wafer;
- placing two points corresponding to the minimum ($t_{eff(min)}$) and maximum ($t_{eff(max)}$) effective time values from the set of previously determined effective time values on the abacus; and
- plotting a straight line between the two points.

6. The method according to claim 5, wherein the minimum and maximum effective time values ($t_{eff(min)}$, $t_{eff(max)}$) are associated with original positions (h) equal to 5 % and 85 % of the total height of the ingot.

**FIG. 1**

Mesurer la concentration en oxygène interstitiel [O$_i$] de la plaquette — F1

Mesurer la concentration en donneurs thermiques [DT$_i$] formés dans la plaquette pendant la solidification du lingot — F2

Déterminer la durée effective t$_{eff}$ du traitement thermique subi par la plaquette pour générer des donneurs thermiques en concentration [DT$_i$] à partir d'oxygène interstitiel en concentration [O$_i$] — F3

Déterminer la position h de la plaquette dans son lingot à partir de la durée effective t$_{eff}$ — F4

**FIG. 2**

$h = -3{,}92.10^{-3} * t_{eff} + 0{,}921$

t$_{eff}$ (min)

**FIG. 3**

F1

Mesurer la résistivité initiale $\rho_1$ dans une zone de la plaquette — F10

Soumettre la plaquette à un recuit à une température comprise entre 350 °C et 550 °C, par exemple 450 °C — F11

Mesurer la résistivité $\rho_2$ dans la zone de la plaquette — F12

Déterminer la concentration en donneurs thermiques $[DT]_{450}$ formés par le recuit à partir des résistivités $\rho_1$ et $\rho_2$ — F13

Déterminer la concentration en oxygène $[O_i]$ de la plaquette à partir de la concentration $[DT]_{450}$ — F14

**FIG. 4**

$\smallint$ ⌐ F2

Mesurer la résistivité initiale $\rho_1$ dans une zone de la plaquette ⟶ F20

Soumettre la plaquette à un recuit à une température supérieure ou égale à 600 °C ⟶ F21

Mesurer la résistivité $\rho_3$ dans la zone de la plaquette ⟶ F22

Déterminer la concentration en donneurs thermiques $[DT_i]$ formés par le traitement thermique à partir des résistivités $\rho_1$ et $\rho_3$ ⟶ F23

**FIG. 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2001076981 B

- FR 2964459

**Littérature non-brevet citée dans la description**

- **WIJARANAKULA C.A. et al.** Formation kinetics of oxygen thermal donors in silicon. *Appl. Phys. Lett.,* 1991, vol. 59 (13), 1608

- **LONDOS C.A. et al.** Effect of oxygen concentration on the kinetics of thermal donor formation in silicon at temperatures between 350 and 500 °C. *Appl. Phys. Lett.,* 1993, vol. 62 (13), 1525